# EUROPEAN PATENT APPLICATION

(11) **EP 2 186 825 A1**
(43) Date of publication of application: **19.05.2010**
(21) Application number: 08019874.0
(22) Date of filing: 13.11.2008
(51) Int. Cl.: C07K 14/705

(54) **Human-derived T cell receptors**

(71) Applicant: Max-Delbrück-Centrum für Molekulare Medizin (MDC), 13092 Berlin (DE)
(72) Inventor: The designation of the inventor has not yet been filed
(74) Representative: Krauss, Jan

(57) **Abstract**

The invention pertains to a human-derived T cell receptor (mhTCR) that, compared to the human wild-type T cell receptor (wtTCR), bears mutations at certain sites of the constant TCR beta region 2 (Cβ2) or the constant TCR beta region 1 (Cβ1) and the constant TCR alpha region (Cα). The presence of these mutations renders the mhTCR to be expressed in higher numbers on the cell surface compared to the wtTCR. Furthermore, the mhTCR of the invention shows a low immunogenic effect, since the number of mutations is low.

SEQUENCE LISTING

<110> Max-Delbrück-Centrum für molekulare Medizin (MDC)
      Berlin-Buch

<120> Human-derived T cell receptors

<130> FBP20892

<160> 7

<170> PatentIn version 3.3

<210> 1
<211> 178
<212> PRT
<213> Homo sapiens

<400> 1

Asp Leu Lys Asn Val Phe Pro Pro Glu Val Ala Val Phe Glu Pro Ser
1               5                   10                  15

Glu Ala Glu Ile Ser His Thr Gln Lys Ala Thr Leu Val Cys Leu Ala
            20                  25                  30

Thr Gly Phe Tyr Pro Asp His Val Glu Leu Ser Trp Trp Val Asn Gly
        35                  40                  45

Lys Glu Val His Ser Gly Val Ser Thr Asp Pro Gln Pro Leu Lys Glu
    50                  55                  60

Gln Pro Ala Leu Asn Asp Ser Arg Tyr Cys Leu Ser Ser Arg Leu Arg
65                  70                  75                  80

Val Ser Ala Thr Phe Trp Gln Asn Pro Arg Asn His Phe Arg Cys Gln
                85                  90                  95

Val Gln Phe Tyr Gly Leu Ser Glu Asn Asp Glu Trp Thr Gln Asp Arg
            100                 105                 110

Ala Lys Pro Val Thr Gln Ile Val Ser Ala Glu Ala Trp Gly Arg Ala
        115                 120                 125

Asp Cys Gly Phe Thr Ser Glu Ser Tyr Gln Gln Gly Val Leu Ser Ala
    130                 135                 140

Thr Ile Leu Tyr Glu Ile Leu Leu Gly Lys Ala Thr Leu Tyr Ala Val
145                 150                 155                 160

Leu Val Ser Ala Leu Val Leu Met Ala Met Val Lys Arg Lys Asp Ser
                165                 170                 175

Arg Gly

<210>  2
<211>  176
<212>  PRT
<213>  Homo sapiens

<400>  2

Asp Leu Asn Lys Val Phe Pro Pro Glu Val Ala Val Phe Glu Pro Ser
1               5                   10                  15

Glu Ala Glu Ile Ser His Thr Gln Lys Ala Thr Leu Val Cys Leu Ala
            20                  25                  30

Thr Gly Phe Phe Pro Asp His Val Glu Leu Ser Trp Trp Val Asn Gly
        35                  40                  45

Lys Glu Val His Ser Gly Val Ser Thr Asp Pro Gln Pro Leu Lys Glu
    50                  55                  60

Gln Pro Ala Leu Asn Asp Ser Arg Tyr Cys Leu Ser Ser Arg Leu Arg
65                  70                  75                  80

Val Ser Ala Thr Phe Trp Gln Asn Pro Arg Asn His Phe Arg Cys Gln
                85                  90                  95

Val Gln Phe Tyr Gly Leu Ser Glu Asn Asp Glu Trp Thr Gln Asp Arg
            100                 105                 110

Ala Lys Pro Val Thr Gln Ile Val Ser Ala Glu Ala Trp Gly Arg Ala
        115                 120                 125

Asp Cys Gly Phe Thr Ser Val Ser Tyr Gln Gln Gly Val Leu Ser Ala
    130                 135                 140

Thr Ile Leu Tyr Glu Ile Leu Leu Gly Lys Ala Thr Leu Tyr Ala Val
145                 150                 155                 160

Leu Val Ser Ala Leu Val Leu Met Ala Met Val Lys Arg Lys Asp Phe
                165                 170                 175

<210>  3
<211>  140
<212>  PRT
<213>  Homo sapiens

<400>  3

Ile Gln Asn Pro Asp Pro Ala Val Tyr Gln Leu Arg Asp Ser Lys Ser
1               5                   10                  15

Ser Asp Lys Ser Val Cys Leu Phe Thr Asp Phe Asp Ser Gln Thr Asn
            20                  25                  30

Val Ser Gln Ser Lys Asp Ser Asp Val Tyr Ile Thr Asp Lys Thr Val
        35                  40                  45

Leu Asp Met Arg Ser Met Asp Phe Lys Ser Asn Ser Ala Val Ala Trp
    50                  55                  60

Ser Asn Lys Ser Asp Phe Ala Cys Ala Asn Ala Phe Asn Asn Ser Ile
65                  70                  75                  80

Ile Pro Glu Asp Thr Phe Phe Pro Ser Pro Glu Ser Ser Cys Asp Val
                85                  90                  95

Lys Leu Val Glu Lys Ser Phe Glu Thr Asp Thr Asn Leu Asn Phe Gln
            100                 105                 110

Asn Leu Ser Val Ile Gly Phe Arg Ile Leu Leu Leu Lys Val Ala Gly
        115                 120                 125

Phe Asn Leu Leu Met Thr Leu Arg Leu Trp Ser Ser
    130                 135                 140

<210>  4
<211>  531
<212>  DNA
<213>  Homo sapiens

<400>  4
gacctgaaca aggtgttccc acccgaggtc gctgtgtttg agccatcaga agcagagatc      60

tcccacaccc aaaaggccac actggtgtgc ctggccacag gcttcttccc cgaccacgtg 120

gagctgagct ggtgggtgaa tgggaaggag gtgcacagtg gggtcagcac agacccgcag 180

cccctcaagg agcagcccgc cctcaatgac tccagatact gcctgagcag ccgcctgagg 240

gtctcggcca ccttctggca gaacccccgc aaccacttcc gctgtcaagt ccagttctac 300

gggctctcgg agaatgacga gtggacccag gatagggcca aacccgtcac ccagatcgtc 360

agcgccgagg cctggggtag agcagactgt ggctttacct cggtgtccta ccagcaaggg 420

gtcctgtctg ccaccatcct ctatgagatc ctgctaggga aggccaccct gtatgctgtg 480

ctggtcagcg cccttgtgtt gatggccatg gtcaagagaa aggatttctg a 531

<210> 5
<211> 537
<212> DNA
<213> Homo sapiens

<400> 5
gacctgaaaa acgtgttccc acccgaggtc gctgtgtttg agccatcaga agcagagatc 60

tcccacaccc aaaaggccac actggtatgc ctggccacag gcttctaccc cgaccacgtg 120

gagctgagct ggtgggtgaa tgggaaggag gtgcacagtg gggtcagcac agacccgcag 180

cccctcaagg agcagcccgc cctcaatgac tccagatact gcctgagcag ccgcctgagg 240

gtctcggcca ccttctggca gaacccccgc aaccacttcc gctgtcaagt ccagttctac 300

gggctctcgg agaatgacga gtggacccag gatagggcca aacccgtcac ccagatcgtc 360

agcgccgagg cctggggtag agcagactgt ggcttcacct ccgagtctta ccagcaaggg 420

gtcctgtctg ccaccatcct ctatgagatc ttgctaggga aggccacctt gtatgccgtg 480

ctggtcagtg ccctcgtgct gatggccatg gtcaagagaa aggattccag aggctag 537

<210> 6
<211> 423
<212> DNA
<213> Homo sapiens

<400> 6
atccagaacc ctgaccctgc cgtgtaccag ctgagagact ctaaatccag tgacaagtct 60

gtctgcctat tcaccgattt tgattctcaa acaaatgtgt cacaaagtaa ggattctgat 120

gtgtatatca cagacaaaac tgtgctagac atgaggtcta tggacttcaa gagcaacagt 180

gctgtggcct ggagcaacaa atctgacttt gcatgtgcaa acgccttcaa caacagcatt 240

attccagaag acaccttctt ccccagccca gaaagttcct gtgatgtcaa gctggtcgag 300

aaaagctttg aaacagatac gaacctaaac tttcaaaacc tgtcagtgat tgggttccga 360

atcctcctcc tgaaagtggc cgggtttaat ctgctcatga cgctgcggct gtggtccagc 420

tga 423

<210> 7
<211> 537
<212> DNA
<213> Artificial

<220>
<223> mhTCR

<400> 7
gacctgaaaa acgtgttccc acccgaggtc gctgtgtttg agccatcaaa agcagagatc 60

gcccacaccc aaaaggccac actggtatgc ctggccacag gcttctaccc cgaccacgtg 120

gagctgagct ggtgggtgaa tgggaaggag gtgcacagtg gggtcagcac agacccgcag 180

cccctcaagg agcagcccgc cctcaatgac tccagatact gcctgagcag ccgcctgagg 240

gtctcggcca ccttctggca gaacccccgc aaccacttcc gctgtcaagt ccagttctac 300

gggctctcgg agaatgacga gtggacccag gatagggcca aacccgtcac ccagatcgtc 360

agcgccgagg cctggggtag agcagactgt ggcatcacct ccgcgtctta ccaccaaggg 420

gtcctgtctg ccaccatcct ctatgagatc ttgctaggga aggccacctt gtatgccgtg 480

ctggtcagtg ccctcgtgct gatggccatg gtcaagagaa aggattccag aggctag 537

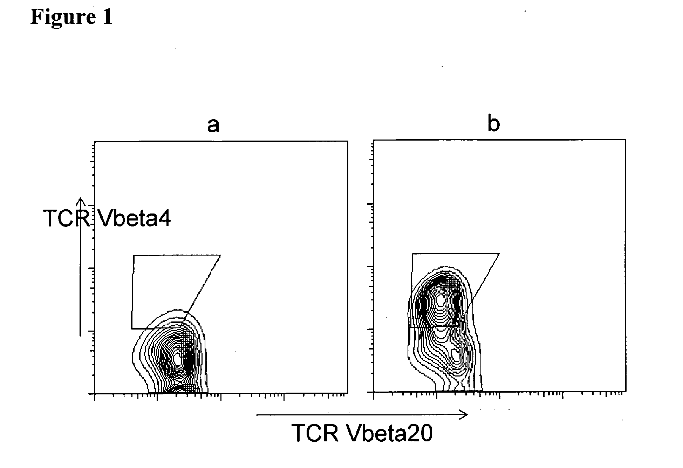

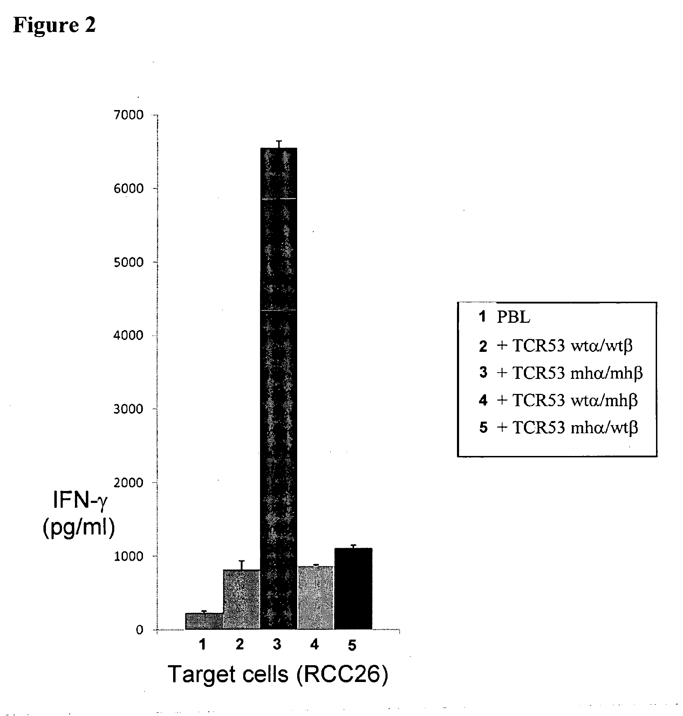

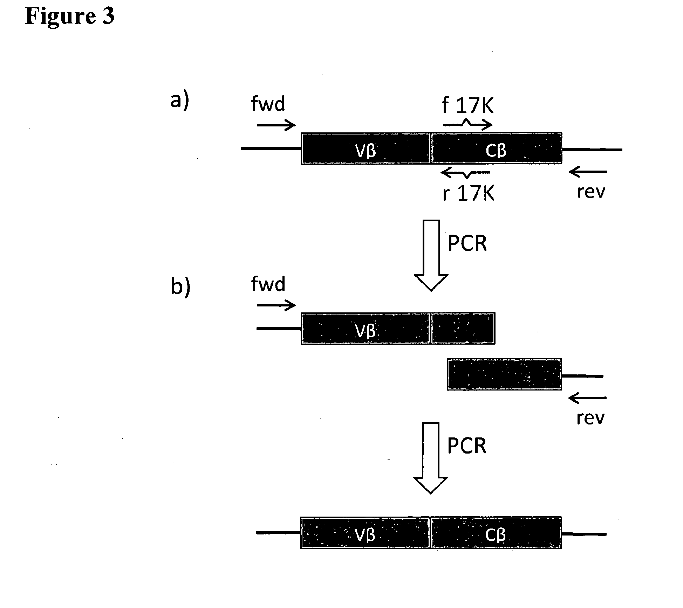

## Description

The invention refers to a human-derived, that is a mutated human T cell receptor (mhTCR) that, compared to the human wild-type T cell receptor (wtTCR), bears mutations at certain sites of the constant TCR beta region 1 (Cβ1) or the constant TCR beta region 2 (Cβ2) and at certain sites of the constant TCR alpha region (Cα). The presence of these mutations renders the mhTCR to be expressed in higher numbers on the cell surface of a T cell compared to the wtTCR, resulting (i) in a T cell with increased avidity to the peptide antigen recognized by the mhTCR, and (ii) in the reduction of the formation of mispaired TCRs consisting of a mixture of an endogenous TCR chain together with a transferred TCR chain (chimaeras). Furthermore, the mhTCR of the invention shows only a low immunogenic effect, since the number of mutations is low.

### Introduction

The T cell receptor (TCR) is a molecule expressed on the surface of T cells. In humans, the TCR has a role in recognizing complexes consisting of antigenic peptides bound to human leukocyte antigen (HLA) molecules. Engagement of the TCR with an HLA-associated antigen ultimately results in the immunological activation of the T cell.

In most T cells, the TCR is a heterodimer consisting of an alpha (α)-chain and a beta (β)-chain. Of the β-chain, two different isoforms exist in humans, namely the Cβ2 that occurs in 80 % of human T cells, and the less frequent Cβ1 (20 %). Each chain of the TCR possesses an N-terminal immunoglobulin (Ig)-like variable (V) domain, an Ig-like constant (C) domain composed of a transmembrane region and a short cytoplasmic tail at the C-terminus.

In the past, TCR gene transfer has been used to produce T cells with new antigen specificity for adoptive therapy. However, the expression of two different TCRs (endogenous and transferred) in T cells has been shown to impair the function of the transferred TCRs or cause unwanted effects through mispaired TCRs (chimaeras) that may result from the simultaneously expressed different TCR chains.

### Description of the invention

Accordingly, the problem underlying the present invention was to provide a TCR with improved properties that would be preferentially expressed in a T cell over an endogenous wtTCR.

This problem is solved by providing a human-derived, i.e. mutated T cell receptor (mhTCR) with a human α-chain and a human β-chain of the TCR bearing mutations at certain positions as described herein. More specifically, the mhTCR of the invention comprises an α-chain and a β-chain with at least one mutation in the constant region of each the α-chain and the β-chain.

### Mutated human T cell receptor (mhTCR)

According to the invention, the β-chin of the mhTCR comprises a human constant beta region 1 (Cβ1) or a human constant beta region 2 (Cβ2). In either case, the mhTCR human constant beta region (1 or 2) comprises a basic amino acid at position 17. This basic amino acid is selected from the group consisting of lysine (K) and arginine (R). The term "position 17" refers to the 17^{th} amino acid residue in the Sequence of SEQ ID NO 1 and SEQ ID NO 2. Besides this mutation at position 17, the Cβ1 or the Cβ2 of the β-chain of the mhTCR may comprise further mutations with the limitation that the function of the TCR is not lost. The term "function of the TCR" is meant to refer to the ability of a TCR to specifically bind to a given antigen, e.g. with a particular affinity.

It is preferred that the sequence of the Cβ2 is at least 75 % identical to the sequence of SEQ ID NO 1 as shown below. More preferably, the sequence of the Cβ2 is at least 80 %, 85 %, 90 % and most preferably 91 % identical to the sequence of SEQ ID NO 1. Moreover, it is preferred that the sequence of the Cβ1 is at least 75 % identical to the sequence of SEQ ID NO 2 shown below. More preferably, the sequence of the Cβ1 is at least 80 %, 85 %, 90 % and most preferably 91 % identical to the sequence of SEQ ID NO 2.

The Cα of the α-chain of the mhTCR comprises according to the invention a serine (S) at position 90, an aspartate (D) at position 91, a valine (V) at position 92, and/or a proline (P) at position 93. It is preferred that all of these mutations are at least present in the Cα of the α-chain of the mhTCR. It is possible that the Cα of the α-chain of the mhTCR may comprise further mutations with the limitation that the function of the TCR is not lost.

It is preferred that the sequence of the Cα is at least 75 % identical to the sequence of SEQ ID NO 3 as shown below. More preferably, the sequence of the Cα is at least 80 %, 85 %, 90 % and most preferably 94 % identical to the sequence of SEQ ID NO 3.

These mutations render the mhTCR to be expressed more strongly than the respective wtTCR and possibly the endogenous TCR. In an embodiment in which the mhTCR comprises a variable region that is configured to bind to a certain antigen, an exchange of antigen specificity of a cell expressing the mhTCR will result. Therefore, the mhTCR of the invention can advantageously be used e.g. in adoptive T cell therapy. Furthermore, the occurrence of chimaeras is significantly reduced, because the α-chain and the β-chain with the mutations described above and herein preferably form mhTCRs with each other.

In a preferred embodiment, the mhTCR comprises at least one further mutation in the Cβ1 or Cβ2. This mutation is selected from the group consisting of an alanine (A) at position 21, an isoleucine (I) at position 132, an alanine (A) at position 135, and a histidine (H) at position 138, wherein the positions mentioned are those in the sequence of SEQ ID NOs 1 (for Cβ2) and 2 (for Cβ1).

The protein sequences for both Cβ2 (SEQ ID NO 1) and Cβ1 (SEQ ID NO 2) are known and are listed below. Differences between the sequences of Cβ2 and Cβ1 are underlined.

### Human constant TCR β-region 2 (Cβ2)

DLKNVFPPEVAVFEPSEAEISHTQKATLVCLATGFYPDHVELSWWVNGKEVHSGVST
DPQPLKEQPALNDSRYCLSSRLRVSATFWQNPRNHFRCQVQFYGLSENDEWTQDRA
KPVTQIVSAEAWGRADCGFTSESYQQGVLSATILYEILLGKATLYAVLVSALVLMAM
VKRKDSRG (SEQ ID NO 1)

### Human constant TCR β-region 1 (Cβ1)

DLNKVFPPEVAVFEPSEAEISHTQKATLVCLATGFFPDHVELSWWVNGKEVHSGVST
DPQPLKEQPALNDSRYCLSSRLRVSATFWQNPRNHFRCQVQFYGLSENDEWTQDRA

KPVTQIVSAEAWGRADCGFTSVSYQQGVLSATILYEILLGKATLYAVLVSALVL-
MAMVKRKDF (SEQ ID NO 2)

The mutation sites described herein are located at positions 17, 21, 132, 135, and 138 of SEQ ID NOs 1 and 2 and are written in bold in the sequence shown above. The mutation sites in Cβ2 and Cβ1 are identical in their positions. The only difference between the two sequences is that at position 135, a glutamic acid (E) is present in Cβ2, whereas a valine is present at position 135 in Cβ1. The position and amino acid substitution for each mutation according to the invention is summarized in tables 1 and 2 below.

**Table 1: Mutations according to the invention in the human constant TCR β-region 2 (Cβ2) of the mhTCR**

| Position (within SEQ ID NO 1) | Amino acid in wild-type | Mutation |
|---|---|---|
| 17 | E | K or R |
| 21 | S | A |
| 132 | F | I |
| 135 | E | A |
| 138 | Q | H |

**Table 2: Mutations according to the invention in the human constant TCR β-region 1 (Cβ1) of the mhTCR**

| Position (within SEQ ID NO 2) | Amino acid in wild-type | Mutation |
|---|---|---|
| 17 | E | K or R |
| 21 | S | A |
| 132 | F | I |
| 135 - | V | A |
| 138 | Q | H |

The protein sequence for Cα (SEQ ID NO 3) is known and is listed below.

### Human constant TCR α-region (Cα)

IQNPDPAVYQLRDSKSSDKSVCLFTDFDSQTNVSQSKDSDVYITDKTVLDMRSMDFK
SNSAVAWSNKSDFACANAFNNSIIPEDTFFPSPESSCDVKLVEKSFETDTNLNFQNLSV
IGFRILLLKVAGFNLLMTLRLWSS (SEQ ID NO 3)

The mutation sites in Cα are located at positions 90, 91, 92, and 93 of SEQ ID NO 3 and are written in bold in the sequence shown above. The mutations according to the invention are also summarized in table 3 below.

**Table 3: Mutations according to the invention in the human constant TCR α-region (Cα) of the mhTCR**

| Position (within SEQ ID NO 3) | Amino acid in wild-type | Mutation |
|---|---|---|
| 90 | P | S |
| 91 | E | D |
| 92 | S | V |
| 93 | S | P |

In addition to the amino acid substitutions given in tables 1 to 3, further substitutions are possible. Specifically, instead of the basic amino acid that is listed in tables 1 to 3 under "Mutation", an equivalent amino acid can also be used in the same position. Equivalence of amino acids is based on similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. Which amino acids are equivalent can be determined by a person of skill in the art based on the information provided herein.

### Nucleic acid molecules for expressing a mhTCR

The problem underlying the present invention is also solved by providing a nucleic acid molecule for expressing an mhTCR as described herein. The nucleic acid molecule can be chosen from the group consisting of DNA molecules, RNA molecules, and artificial nucleic acids including peptide nucleic acids (PNA), Morpholino and locked nucleic acids (LNA), glycol nucleic acids (GNA) and threose nucleic acids (TNA), as well as mixtures thereof.

This nucleic acid encodes for a mhTCR as described above and herein. Specifically, the nucleic acid encodes for a β-chain of an mhTCR and/or for an α-chain of a mhTCR. Since the mhTCR consists of both an α-chain and a β-chain, which are separately encoded, the invention also pertains to a nucleic acid that encodes for each of the α-chain and the β-chain separately, but in each case the nucleic acid is suitable for expressing a mhTCR as described herein in a cell. Moreover, the invention pertains to the use of a nucleic acid encoding for a β-chain of an mhTCR and/or for an α-chain of a mhTCR for expressing a mhTCR in a cell. When the α-chain and a β-chain are expressed separately, the mhTCR is formed in the cell in which the α-chain and the β-chain are expressed. In addition, the invention pertains to a nucleic acid that encodes for both the α-chain and the β-chain together, i.e. encodes for the mhTCR as described herein.

The β-chain of the mhTCR that the nucleic acid molecule encodes for comprises a Cβ1 or a Cβ2. In either case, the Cβ1 or the Cβ2 comprises a basic amino acid at position 17 that is selected from the group consisting of lysine (K) and arginine (R). In the case of Cβ1, the sequence of the Cβ1 encoded by the nucleic acid is at least 75 % identical to the sequence of SEQ ID NO 2. In the case of Cβ2, the sequence of the Cβ2 encoded by the nucleic acid is at least 75 % identical to the sequence of SEQ ID NO 1. In both cases, i.e. for Cβ2 and Cβ1, it is more preferred that the sequence of the Cβ2 is at least 80 %, 85 %, 90 % and most preferably 91 % identical to the sequence of SEQ ID NO 1 or SEQ ID NO 2, respectively.

The nucleic acid molecule may further encode e.g. for the variable part of the β-chain of the mhTCR. In such a case, the nucleidc acid does not only comprise a first sequence portion for encoding for a β-chain of an mhTCR and/or for an α-chain of a mhTCR, but comprises further a second sequence portion for encoding e.g. for the variable part of the β-chain of the mhTCR or another protein, polypeptide or tag.

The α-chain of the mhTCR that the nucleic acid molecule-encodes for a Cα which comprises a serine (S) at position 90, an aspartate (D) at position 91, a valine (V) at position 92, and/or a proline (P) at position 93. Preferably, the nucleic acid molecule encodes for a Cα that comprises all of these four mutations. Preferably, the sequence of the Cα encoded by the nucleic acid molecule is at least 75 % identical to the sequence of SEQ ID NO 3. More preferably, the sequence of the Cα is at least 80 %, 85 %, 90 % and most preferably 94 % identical to the sequence of SEQ ID NO 3.

Due to the degeneracy of the genetic code, several nucleic acid molecules can encode for Cβ1, Cβ2, and/or Cα with the mutation or mutations as described herein. Below, the DNA sequences encoding for Cβ1 (SEQ ID NO 4), Cβ2 (SEQ ID NO 5), and Cα (SEQ ID NO 6), all as wild-type, are provided. In each case, the codon encoding for an amino acid residue that may be mutated according to the invention in the mhTCR is shown in bold.

### Nucleotide sequence (DNA) encoding for Cβ wild-type

gacctgaacaaggtgttcccacccgaggtcgctgtgtttgagccatcagaagcagagatctcccacacccaaaaggccacactggtgt
gcctggccacaggcttcttccccgaccacgtggagctgagctggtgggtgaatgggaaggaggtgcacagtggggtcagcacagac
ccgcagcccctcaaggagcagcccgccctcaatgactccagatactgcctgagcagccgcctgagggtctcggccaccttctggcag
aacccccgcaaccacttccgctgtcaagtccagttctacgggctctcggagaatgacgagtggacccaggatagggccaaacccgtc
acccagatcgtcagcgccgaggcctggggtagagcagactgtggctttacctcggtgtcctaccagcaaggggtcctgtctgccacc
atcctctatgagatcctgctagggaaggccaccctgtatgctgtgctggtcagcgcccttgtgttgatggccatggtcaagagaaaggat
ttctga (SEQ ID NO 4)

### Nucleotide sequence (DNA) encoding for Cβ2 wild-type

gacctgaaaaacgtgttcccacccgaggtcgctgtgtttgagccatcagaagcagagatctcccacacccaaaaggccacactggtat
gcctggccacaggcttctaccccgaccacgtggagctgagctggtgggtgaatgggaaggaggtgcacagtggggtcagcacaga
cccgcagcccctcaaggagcagcccgccctcaatgactccagatactgcctgagcagccgcctgagggtctcggccaccttctggca
gaacccccgcaaccacttccgctgtcaagtccagttctacgggctctcggagaatgacgagtggacccaggatagggccaaacccgt
cacccagatcgtcagcgccgaggcctggggtagagcagactgtggcttcacctccgagtcttaccagcaaggggtcctgtctgccac
catcctctatgagatcttgctagggaaggccaccttgtatgccgtgctggtcagtgccctcgtgctgatggccatggtcaagagaaagg
attccagaggctag (SEQ ID NO 5)

### Nucleotide sequence (DNA) encoding for Cα

atccagaaccctgaccctgccgtgtaccagctgagagactctaaatccagtgacaagtctgtctgcctattcaccgattttgattctcaaac
aaatgtgtcacaaagtaaggattctgatgtgtatatcacagacaaaactgtgctagacatgaggtctatggacttcaagagcaacagtgct
gtggcctggagcaacaaatctgactttgcatgtgcaaacgccttcaacaacagcattattccagaagacaccttcttccccagcccaga
aagttcctgtgatgtcaagctggtcgagaaaagctttgaaacagatacgaacctaaactttcaaaacctgtcagtgattgggttccgaatc
ctcctcctgaaagtggccgggtttaatctgctcatgacgctgcggctgtggtccagctga (SEQ ID NO 6)

Tables 4 to 6 summarize the nucleic acid codons that are used in the wild-type sequence of Cβ2, Cβ1, and Cα for encoding amino acids at positions that are relevant according to the invention. For each wild-type codon, possible codons for mutations are listed.

**Table 4: Mutations according to the invention in the nucleic acid encoding for the human constant TCR β-region 2 (Cβ2) of the mhTCR**

| Position (within SEQ ID NO 1) | Nucleic acid codon in wild-type | Possible Mutations |
|---|---|---|
| 17 | gaa | aaa or aag (for K), or cga, cgc, cgg, cgt, aga, or agg (for R) |
| 21 | tcc | gca, gcc, gcg, or gct (for A) |
| 132 | ttc | ata, atc, or att (for I) |
| 135 | gag | gca, gcc, gcg, or gct (for A) |
| 138 | cag | cac, or cat (for H) |

**Table 5: Mutations according to the invention in the nucleic acid encoding for the human constant TCR β-region 1 (Cβ1) of the mhTCR**

| Position (within SEQ ID NO 2) | Nucleic acid codon in wild-type | Possible Mutations |
|---|---|---|
| 17 | gaa | aaa or aag (for K), or cga, cgc, cgg, cgt, aga, or agg (for R) |
| 21 | tcc | gca, gcc, gcg, or gct (for A) |
| 132 | ttc | ata, atc, or att (for I) |
| 135 | gtg | gca, gcc, gcg, or gct (for A) |
| 138 | cag | cac, or cat (for H) |

**Table 6: Mutations according to the invention in the nucleic acid encoding for the human constant TCR α-region (Cα) of the mhTCR**

| Position (within SEQ ID NO 3) | Nucleic acid codon in wild-type | Possible Mutations |
|---|---|---|
| 90 | cca | tca, tcc, tcg, tct, agc, or agt (for S) |
| 91 | gaa | gac, or gat (for D) |
| 92 | agt | gta, gtc, gtg, or gtt (for V) |
| 93 | tcc | cca, ccc, ccg, or cct (for P) |

Accordingly, a nucleic acid encoding for a Cβ2 in which all four positions of interest are mutated (namely position 17 E>K, position 21 S>A, position 132 F>I, position 135 E>A, and position 138 Q>H) could, for example, be encoded by the following nucleic acid molecule (mutated codons are shown in bold) (SEQ ID NO 7):

gacctgaaaaacgtgttcccacccgaggtcgctgtgtttgagccatcaaaagcagagatcgcccacacccaaaaggccacactggta
tgcctggccacaggcttctaccccgaccacgtggagctgagctggtgggtgaatggaaggaggtgcacagtggggtcagcacaga
cccgcagcccctcaaggagcagcccgccctcaatgactccagatactgcctgagcagccgcctgagggtctcggccaccttctggca
gaacccccgcaaccacttccgctgtcaagtccagttctacgggctctcggagaatgacgagtggacccaggatagggccaaacccgt
cacccagatcgtcagcgccgaggcctggggtagagcagactgtggcatcacctccgcgtcttaccaccaaggggtcctgtctgccac
catcctctatgagatcttgctagggaaggccaccttgtatgccgtgctggtcagtgccctcgtgctgatggccatggtcaagagaaagg
attccagaggctag

A person of skill in the art will be able to devise other nucleic acid molecules that encode for a β-chain of an mhTCR and/or for an α-chain of a mhTCR based on the information provided herein together with his general knowledge.

### Expression vector

In a further aspect, the invention relates to an expression vector comprising a nucleic acid as described above and herein for expressing a mhTCR. The expression vector can be a plasmid, including retroviral plasmids, a cosmid, phage, or alike. In the expression vector, the encoding nucleic acid is preferably operably linked to a promoter for initiating the transcription of the nucleic acid encoding Cβ1, Cβ2 and/or Cα.

### Host cell

In yet a further aspect, the invention relates to a host cell containing a nucleic acid molecule as described above and herein or an expression vector as described above and herein. The host is preferably a mammalian cell, in particular a human cell. Such a host cell is e.g. obtainable by introducing a vector as described above into a cell using known methods, such as physical methods (e.g. electroporation, microinjection), chemical methods (e.g. calcium phosphate, lipofection), or virus-mediated methods (using viruses or parts thereof).

### Use of an expression vector

In addition, the invention relates to the use of a nucleic acid and/or of an expression vector as described above and herein for expressing a mhTCR in a cell. The cell is preferably a mammalian cell, in particular a human cell.

### Pharmaceutical composition

In a further aspect, the invention relates to a pharmaceutical composition. This pharmaceutical composition may comprise a mhTCR as described above and herein, a nucleic acid molecule for expressing a mhTCR as described above and herein and/or an expression vector for expressing a mhTCR as described above and herein. Optionally the pharmaceutical composition may further comprise a pharmaceutically suitable carrier as known in the art.

### Kit

In yet a further aspect, the invention relates to a kit comprising a mhTCR as described above and herein, a nucleic acid molecule as described above and herein, an expression vector for expressing a mhTCR as described above and herein, a host cell as described above and herein and/or a pharmaceutical composition as described above and herein.

### Method for treating a subject

In addition, the invention relates to a method for treating a subject in need of treatment (i.e. a patient) with an mhTCR (i.e. through adoptive therapy). Such a method comprises introducing an mhTCR and/or a nucleic acid molecule as described above and herein and/or an expression vector as described above and herein into a cell, and administering the cell to the patient. Such a method is referred to by a person of skill in the art as "adoptive cell therapy" (ACT).

### Method for expressing an mhTCR

Furthermore, the invention relates to a method of expressing an mhTCR as described above and herein in a cell. The method comprises the steps of (a) providing a cell, and (b) introducing into the cell an mhTCR as described above and herein, a nucleic acid molecule as described above and herein, and/or an expression vector as described above and herein. The cell that is provided can be an isolated cell, or can be a cell that is part of a tissue, organ or organism. Accordingly, the method can be an in vitro method in one embodiment and an in vivo method in another embodiment.

In a preferred embodiment, the present invention does not concern a process for cloning human beings, a process for modifying the germ line genetic identity of human beings, uses of human embryos for industrial or commercial purposes, processes for modifying the genetic identity of animals which are likely to cause them suffering without any substantial medical benefit to man or animal, or animals resulting from such processes.

### Figures

Figure 1:
   The TCR-deficient T cell line Jurkat76 was transduced with a renal cell carcinoma-specific TCR (TCR26, Vbeta 20) and enriched for TCR-expressing cells, resulting in the indicator cell line J76/TZR26. This cell line was used for the expression of different mutated constructs of a second TCR, which recognizes the tumor-associated antigen NY-ESO-1. The expression level of the NY-ESO-1-TCRs (and thereby the displacement of the endogenous TCR26) was determined using an antibody that recognizes the variable region of the beta chain of the NY-ESO-1-TCR (TCR Vbeta 4). Whereas the NY-ESO-1 wtTCR (a) was not expressed in the indicator cell line, the construct with a 9 amino acid mutation (NY-ESO-1 mhTCR) was expressed on the cell surface (b). The 9 amino acid mutations were those shown in tables 1 (with a K in position 17) and 3.
Figure 2:
   Peripheral blood lymphocytes (PBL) were transduced with different variants of the renal cell carcinoma-reactive TCR53. Transduced PBL were co-cultivated with a target cell line expressing the antigen recognized by TCR53. After 24 hours, the Interferon-γ (IFN-γ) concentration in cell culture supernatant was analyzed by INF-γ-ELISA.
Figure 3:
   To introduce mutations at certain positions of the human constant TCR regions, two successive PCR steps were performed. The TCR gene was divided into two parts, the first part consisted of the variable region and the constant region up to a few nucleotides behind the mutation, the second part started shortly in front of the mutation and involved the rest of the constant region.
   (a) The first part was amplified using a forward primer binding to the plasmid directly in front of the TCR gene (fwd) and a reverse primer of approximately 20 nucleotide length including the mutation (e.g. r 17K: ATCTCTGC***TTT***TGATGGCTC, SEQ ID NO 8). The second part was amplified using a forward primer complementary to the reverse primer used for the first part (e.g. f 17K: GAGCCATCA***AAA***GCAGAGAT, SEQ ID NO 9) and a reverse primer binding the plasmid directly behind the TCR gene (rev). Codons introducing the mutation are shown in bold.
   (b) The two parts of the new TCR gene were then combined by annealing of the 20 nucleotide complementary sequences and subsequently amplified using the forward primer of the first part (fwd) and the reverse primer of the second part (rev). Finally, the generated new TCR gene was integrated into the retroviral vector MP71-PRE via restriction sites *Not* I and *EcoR* I.

## Claims

1. A human-derived T cell receptor (mhTCR) with
- a β-chain comprising
a human constant beta region 1 (Cβ1) or a human constant beta region 2 (Cβ2) which comprise
- a basic amino acid at position 17 that is selected from the group consisting of lysine (K) and arginine (R),
wherein the sequence of the Cβ2 is at least 75 % identical to the sequence of SEQ ID NO 1 and wherein the sequence of the Cβ1 is at least 75 % identical to the sequence of SEQ ID NO 2; and
- an α-chain comprising
a human constant TCR α-region (Cα) which comprises
- a serine (S) at position 90,
- an aspartate (D) at position 91,
- a valine (V) at position 92, and
- a proline (P) at position 93,
wherein the sequence of the Cα is at least 75 % identical to the sequence of SEQ ID NO 3.

2. The mhTCR according to claim 1, further comprising a mutation in the Cβ1 or Cβ2 that is selected from the group consisting of
- an alanine (A) at position 21,
- an isoleucine (I) at position 132,
- an alanine (A) at position 135, and
- a histidine (H) at position 138.

3. A nucleic acid molecule encoding
- a β-chain of a mhTCR, comprising
a human constant beta region 1 (Cβ1) or a human constant beta region 2 (Cβ2) which comprises
- a basic amino acid at position 17 that is selected from the group consisting of lysine (K) and arginine (R),
wherein the sequence of the Cβ2 encoded by the nucleic acid is at least 75 % identical to the sequence of SEQ ID NO 1 (for Cβ2) or 2 (for Cβ1); and/or
- an α-chain of a mhTCR, comprising
a human constant TCR α-region (Cα) which comprises
- a serine (S) at position 90
- an aspartate (D) at position 91
- a valine (V) at position 92, and
- a proline (P) at position 93,
wherein the sequence of the Cα encoded by the nucleic acid is at least 75 % identical to the sequence of SEQ ID NO 3.

4. An expression vector comprising a nucleic acid according to claim 3 for expressing a mhTCR.

5. A host cell containing a nucleic acid molecule according to claim 3 or an expression vector according to claim 4, in particular a human host cell.

6. Use of an expression vector according to claim 4 for expressing a mhTCR in a cell, in particular in a human cell.

7. A pharmaceutical composition comprising a mhTCR according to claim 1 and/or 2, or a nucleic acid molecule for expressing a mhTCR according to claim 3 and/or an expression vector for expressing a mhTCR according to claim 4, optionally together with a pharmaceutically suitable carrier.

8. A kit comprising a mhTCR according to claim 1 and/or 2 or a nucleic acid molecule according to claim 3 and/or an expression vector for expressing a mhTCR according to claim 4 and/or a host cell according to claim 5 and/or a pharmaceutical composition according to claim 7.

9. A method for treating a subject in need of treatment with a mhTCR comprising introducing a mhTCR according to claim 1 and/or 2 and/or a nucleic acid molecule according to claim 3 and/or an expression vector according to claim 4 into a cell, and administering the cell to a subject.

10. A method for expressing an mhTCR according to claim 1 and/or 2 in a cell comprising
- providing a cell, and
- introducing into the cell a hTCR according to claim 1 or 2, a nucleic acid molecule according to claim 3, and/or an expression vector according to claim 4.
